# EUROPEAN PATENT SPECIFICATION

(11) **EP 0 745 432 B1**
(45) Date of publication and mention of the grant of the patent: **03.05.2000**
(21) Application number: 95108432.6
(22) Date of filing: 01.06.1995
(51) Int. Cl.: B05C 1/16, A61F 13/15

(54) **Continuous adhesive printing onto discontinuous series of disposable absorbent article**
Verfahren zum kontinuierlichen Bedrucken eines Klebstoffs auf eine diskontinuierliche Reihe absorbierender Einwegartikel
Procédé d'impression d'un adhésif sur une série discontinue d'articles absorbents

(43) Date of publication of application: 04.12.1996
(73) Proprietor: THE PROCTER & GAMBLE COMPANY, Cincinnati, Ohio 45202 (US)
(72) Inventor: Hagemeister, Eric Peter, D-74532 Ilshofen (DE); Hundorf, Harald Hermann, D-61352 Bad Homburg (DE); Lender, Horst Andreas, D-91740 Röckingen (DE); Plumley, Julian Ashton, D-74589 Satteldorf (DE)
(74) Representative: Hirsch, Uwe Thomas

(56) References cited:
- EP-A- 0 611 607
- EP-A- 0 621 082
- EP-A- 0 622 126
- WO-A-94/29524
- FR-A- 2 244 670
- GB-A- 2 134 420
- US-A- 4 851 069

## Description

### Field of the invention

The present invention relates to a process for making disposable absorbent articles such as sanitary napkins, panty liners, catamenials, incontinence inserts, and diapers for adults or babies. In particular the present invention relates to a process for making disposable absorbent articles comprising two materials which are adhered to each other. Importantly, the process utilises continuous roll printing to provide the adhesive onto a discontinuous series of separated pieces of a substrate. This adhesive printing process can be used to provide full surface covering adhesive areas without the need to cut through adhesive when separating the pieces of the substrate after application of the adhesive.

### Background of the invention

Absorbent articles such as sanitary napkins, panty liners, catamenials, incontinence inserts and diapers for adults or babies are commonly provided with an adhesive on their garment-facing surface to attach them during their usage period to a garment of the user. In particular sanitary napkins and panty liners are commonly provided with a pressure sensitive, hotmelt, adhesive which attaches to the undergarment of the wearer and thereby improves fit and comfort of the product for the wearer. These adhesives are typically covered with a release paper prior to use.

More generally absorbent articles are provided with adhesive areas in order to combine materials which ultimately make up part or the whole of the absorbent article. In particular, multi-layer structures forming the topsheet, core or backsheet are often combined by adhesives. The combining of the topsheet, the core and the backsheet to each other also can be accomplished by adhesives.

Typically these products are made by high speed machinery. The machinery includes equipment which adds the adhesive in a very fast and efficient manner, ensuring consistency of the absorbent products over large quantities thereof.

A common way of providing an adhesive is a slot-coating or spraying of the adhesive onto a continuously conveyed thread of material. The surface of this material, coated with adhesive, is then joined to another material forming all or part of the disposable absorbent articles. These adhesive application methods allow adhesive application without contact between apparatus and material to be coated. This is desirable in particular for thermoplastic film or nonwoven materials often used in disposable absorbent articles which are very heat sensitive and can be incompatible with adhesives which require high melting temperatures.

An alternative method of adhesive application to material used in absorbent articles is a screen printing method. In the screen printing method an adhesive is applied to the inside of a rotating roll having apertures. This roll, also called screen, is contacted with the continuous conveyed threat of material such that adhesive is transferred through the holes of the material. Again, only the adhesive is heated while the screen surface contacting the surface to which the adhesive is applied is not heated.

Another alternative application method for adhesive is to render one of the materials used in the production of absorbent articles adhesive. This can be done in a separate step independent and long before use of the material in the article. For example adhesive tape or film - as used on baby diapers - have one adhesive surface and one non-adhesive surface. The adhesive surface is not firmly attached (or at least realisably attached) to the non-adhesive surface by winding the tape or film up into a roll. Upon use of the material the roll is then unwound to present the adhesive surface to the material to which it is intended to attach the tape or film.

US 5,064,492 provides in this manner an impermeable film onto which a particular adhesive, which is said to be non-blocking below about 43°C (110°F), is printed. The adhesive is provided to the film by a patterned printing roll and a cooled counter roll or a series of such printing units if patterns in the adhesive are desired. The winding of the adhesive coated film is conducted below the blocking temperature.

This method of providing precoated film adds multiple complexities to logistics, storage and final manufacturing of disposable articles. Even accidental heating of any portion of the precoated roll would render it useless (sticking to itself). Also all benefits of the positioning accuracy of adhesive printing are lost in the alignment difficulties associated with film unwinding. Further the adhesive has to be re-heated to return it to a state of stickiness such that added energy consumption is paired with a probability of melting through the underlying film material. Also this heating of the adhesive causes degradation due to the added heating/cooling cycle.

In parallel patent application entitled "Adhesive printing for disposable absorbent articles" a process for adhesive printing is disclosed where a continuous or pattern configuration of adhesive is provided onto a continuous thread of a first material. In particular for continuous adhesive provided in this fashion the separation of individual pieces of the substrate made by this process requires cutting through an adhesive layer. Similarly GB 2 134 420 and WO 94/29524 describe modified adhesive transfer cylinders to apply adhesive to a continuous web.

One common drawback of most of the above mentioned adhesive application processes is their inflexibility, inaccuracy relative to the shape of the adhesive to be applied and that they essentially can only provide the adhesive continuously which then require to cut through an adhesive layer. On/Off systems for coating or spraying have the drawback of delay in their systems response and generally the problems associated with accelerating and decelerating mass streams. For hollow drum screen printing it is possible to create a pattern in the screen which would allow to create adhesive patterns. However, this is limited in that the screen has to provide a fairly even distribution of adhesive application sites (holes) in order to exclude temperature variations resulting in unstable application processes. Also adhesive screen printing cannot provide an even, full surface adhesive coverage due to the maximum apertured dimensions and total open area of such a screen in respect to its stability.

However, even if systems are used which allow intermittent application of adhesive to a continuous substrate separation of the continuous substrate into individual pieces requires a high degree of accuracy on the relative placement of the adhesive to the separation mechanism (cutting) or it is accepted that the separation mechanism cuts through an adhesive layer. Cutting through an adhesive layer of course has the drawback of added wear and efficiency losses due to increased cleaning requirements for cutting equipment.

The applicants have now found a process of providing adhesive areas to disposable absorbent articles, which areas can extend at least partially to the periphery of the substrate without having to cut through adhesive. The process allows full surface adhesive coverage, and is generally more stable and accurate than prior art processes. The inventive process causes less production down time than a process providing the same adhesive but using conventional adhesive delivery methods.

It is therefore the object of the present invention to provide disposable absorbent articles having an adhesive area which joins two materials comprised in the disposable absorbent article and to provide an accurate and efficient process alternative to current adhesive applications thereby allowing the adhesive area to extend to the periphery of the substrate to which it is applied and which was so far not possible at the production speeds typical for disposable absorbent articles.

### Summary of the invention

This object is achieved according to the invention by the process as defined in claim 1. Particular embodiments of the invention are the subject of the dependent claims.

The present invention relates to a process for providing an adhesive area to a first surface of a substrate for use in a disposable absorbent article. The process steps are
providing a series of separated pieces of the substrate, each of the pieces having an accessible first surface.
conveying the series of separated pieces of the substrate, i.e. not a continuous thread of the substrate, in a machine direction.
providing an adhesive area onto the first surface of each of the pieces of the substrate by continuous roll printing. This way an adhesive area of which at least a portion of its peripheral edge coincides with the peripheral edge of the first surface of the pieces of the substrate is created. Preferably the roll printing provides the whole surface of the first substrate with an adhesive such that the peripheral edge of the adhesive area and the first surface coincide totally.

The roll printing step of the process according to the present invention is preferably conducted by a rotating transport roll for transporting the adhesive. The transport roll continuously receives the adhesive in a receiving portion of its rotation path. The transport roll delivers the adhesive to the first surface in the delivering portion of the rotation path of the transport roll but due to the discontinuous presence of the separated pieces the adhesive is discontinuously delivered.

The adhesive is typically a hot melt adhesive and is applied in a liquefied state typically achieved by melting. Therefore, the receiving portion of the rotation path of the transport roll is typically inside a bath of the molten adhesive.

In one embodiment of the process of the present invention the adhesive area provides a permanent joining means for the separated pieces of the substrate to the remainder of the disposable absorbent article.

In an alternative embodiment of the process of the present invention the disposable absorbent article is used in the undergarment of a user and comprises a panty fastening adhesive for releasably joining the article to the undergarment of the user and this panty fastening adhesive is provided by the process according to the present invention. Typically such disposable absorbent articles are prepared from a continuous substrate which is severed into separate pieces which are then provided with the panty fastening adhesive area. Typically such disposable absorbent articles are sanitary napkins, catamenials or panty liners which comprise a topsheet, a backsheet and an absorbent core between the topsheet and the backsheet. Typically the backsheet provides the first surface onto which the adhesive area is printed by the process according to the present invention.

The panty fastening adhesive typically is covered by a continuous strip of a releasable cover means most often a siliconised release paper which is placed as a continuous strip onto the adhesive area on the separated absorbent articles and is then severed in the gap between the separated absorbent articles to provide the individualised disposable articles. Alternatively the continuous strip of releasable cover means can remain unsevered and be stored for later removal of the absorbent articles from the continuous strip of releasable cover means.

In a possible embodiment of the present process the adhesive is provided to the first surface from below. This ensures drip free adhesive application without the need for additional safeguards to prevent dripping.

It is possible that the process according to the present invention provides the adhesive area in a shape which comprises parts which are neither parallel nor perpendicular to the machine direction and which are preferably non-linear.

The adhesive area provided on the first surface has a peripheral edge which at least partially coincides with the peripheral edge of the surface of the material to which the adhesive area is provided. Preferably the adhesive area is provided to fully cover the surface of the first material such that their peripheral edges fully coincide.

The adhesive area can provide a releasable attachment means such as is typical between a release paper and a panty fastening adhesive. However, the current process is not limited to such adhesive application but can also provide a permanent connection between the first and the second material.

### Brief description of the drawings

Figure 1 shows a schematic view of an embodiment of the process steps of the present invention.

Figure 2 shows a cross-sectional schematic view of the printing equipment used in the adhesive printing step according to the present invention.

Figures 3 - 14 show alternative engravings and their cross-sectional topographies useful for the rotating transport roll according to the present invention.

### Detailed description of the invention

The absorbent article has a body facing surface, typically provided by a liquid permeable substrate of fibrous or film like structure often termed topsheet; a garment facing surface, preferably provided by a liquid impermeable, but breathable substrate often termed backsheet and an absorbent structure placed between the body facing surface and the garment facing surface, typically termed the absorbent core. The absorbent article has a longitudinal axis and a lateral axis and can comprise any of the components or features usual in the art. In particular side wrapping elements, side flap components, or wings as well as any sort of extensibility or elastication feature can be comprised in absorbent articles.

The disposable article for absorbing liquid is described below by reference to a sanitary napkin or panty liner. However products such as adult or baby diaper inserts comprising adhesives can similarly benefit from the process of the present invention.

A preferred sanitary napkin or panty liner made according to the present invention has a pair of side wrapping elements or "undergarment covering components". They provide coverage of the wearer's panties to reduce side soiling (i.e., staining of the edges of the panty crotch) and are typically smaller than conventional flaps or wings.

The function of side wrapping elements, whether integral or joined to the article after being formed separately, is further improved by rendering them extensible in one or both directions parallel to the longitudinal axis and/or lateral axis. The extensibility can be provided across all or only part of the side wrapping elements and can be achieved by pleating or ring-rolling those parts which are to be rendered extensible.

A typical sanitary napkin or panty liner comprises a liquid pervious topsheet, a liquid impervious backsheet joined to the topsheet, and an absorbent core positioned between the topsheet and the backsheet. On the backsheet is provided an adhesive area providing the panty-fastening adhesive which is covered by a release paper or the like prior to use of the article.

The present invention relates to a process for providing a disposable absorbent article comprising a first material and a second material which are joined by adhesive. The materials may be any of the materials typical in the context of disposable absorbent articles. It includes permanent and releasable adhesive surfaces which need to be provided and therefore includes absorbent articles having an adhesive area on their garment facing surface which is covered by a protective cover means such as a release paper.

In the production of absorbent articles several adhesive connections are typically formed all of which, some of which or at least one of which utilises the process according to the present invention. Therefore, disposable absorbent articles generally suitable to benefit from application of the present invention are described below.

The adhesive applied by the process according to the present invention can be any of a series of adhesives. Typically, hot melt adhesives, typically comprising a thermo-plastic base material, in combination with a tackyfying resin, or a mixture of various such materials, can be applied by the present process. Typical hot melt adhesives have a minimum melting temperature of about 80°C, often even about 100°C. The requirement for these hot melt adhesives is of course that they maintain their adhesive performance until disposal of the disposable absorbent article, i.e. during manufacturing, storage, transport and use of the disposable absorbent article. Typically, the highest temperature during these activities is the usage temperature at about 40°C when the disposable absorbent article is used on the body of a human. However, higher temperatures can occur for example when articles are left in a vehicle in the sun, where temperatures of 60°C and higher have been reported.

When applying the adhesive by roll printing according to the present invention it is necessary that the cohesive forces of the adhesive are lower than the adhesive forces to the substrate to which the adhesive is printed and that the cohesive forces within the substrate onto which the adhesive is printed are also higher than the cohesive forces of the adhesive. If this force relation is not met parts of the substrate will disassociate from the substrate and attach to the adhesive surface on the roll printing roll. In order to assure this cannot happen it can be necessary to keep a tight temperature control since the cohesive strength of hot melt adhesives is highly dependent on the temperature of the adhesive. It is understood by those skilled in the art that simple trials will allow to immediately confirm whether the adhesive roll printing to a certain substrate can be performed and at which temperature given a certain adhesive.

In order to more fully assess the utility of the process of the present invention a description of a typical disposable absorbent article follows.

### Topsheet

The topsheet is compliant, soft feeling, and non-irritating to the wearer's skin. Useful topsheets are well known in the art and include especially those disclosed in copending application "Disposable absorbent articles having a shaped panty fastening adhesive."

When referring to the topsheet a multi layer structure or a mono layer structure is contemplated. Joining the topsheet to another portion of the absorbent article or internal joining of multi layer topsheets can be subject to the process of the present invention.

### Absorbent structure

When referring to the absorbent structure a multi layer structure or a mono layer structure are contemplated. Joining the absorbent structure to another portion of the absorbent article or internal joining of multi layer absorbent structures can be subject to the process of the present invention. Useful absorbent structures are well-known in the art and include especially those disclosed in copending application "Disposable absorbent articles having a shaped panty fastening adhesive".

For joining the surface of the absorbent structure or a layer of the absorbent structure to another surface it is important to observe the above remarks in respect to the cohesive and adhesive forces and their relation to each other. In particular absorbent structures of cellulosic fluff material have often been found to not satisfy the requirement of sufficient cohesive strength to allow printing to them. A possible solution in these circumstances is to use the adhesive roll printing process on the surface to which the cellulosic fibrous absorbent structure or layer of the absorbent structure is to be joined.

### Backsheet

The backsheet primarily prevents the exudates absorbed and contained in the absorbent structure from wetting articles that contact the absorbent product such as underpants, pants, pyjamas and undergarments. Useful backsheets are well known in the art and include especially those disclosed in copending application "Disposable absorbent articles having a shaped panty fastening adhesive". When referring to the backsheet a multi layer structure or a mono layer structure is contemplated. Joining the backsheet to another portion of the absorbent article or internal joining of multi layer backsheets can be subject process of the present invention.

### The panty-fastening-adhesive

The backsheet typically forms the garment facing surface of the absorbent article on which the panty fastening adhesive is placed. Panty-fastening-adhesives can comprise any adhesive or glue used in the art for such purposes. These adhesives typically are pressure sensitive and remain tacky well below their application temperature.

Suitable non-extensible adhesives are Savare LA203 and Savare LA303 made by Savare I.C. of Milan in Italy, Coramelt 867 by Koemmerling in Pirmasens in Germany, Fuller D3964ZP and Fuller H-2238ZP manufactured by the H.B. Fuller Co. in Lueneburg, in Germany. Suitable adhesive fasteners are also described in U.S. Patent 4,917,697.

It is a feature of the present invention that the shape of the panty-fastening adhesive area can comprise non-linear portions which are not coincident with the periphery of the garment facing surface of the absorbent article. The panty-fastening adhesive also need not but can be coextensive with the garment facing surface of the sanitary napkin.

Prior to use of the absorbent article the panty fastening adhesive is typically protected from contamination and from sticking to any surface where this is not desired by a protective cover means such as a silicone coated release paper, a plastic film or any other easily removable cover. The protective cover means can be provided as a single piece or in a multitude of pieces e.g. to cover individual adhesive areas. It also can perform other functions such as provide individualised packaging for the article or provide a disposal function.

### Process steps of the present invention

In the following a preferred embodiment of providing the panty fastening adhesive to a sanitary napkin according to the present invention will be described with reference to the drawings.

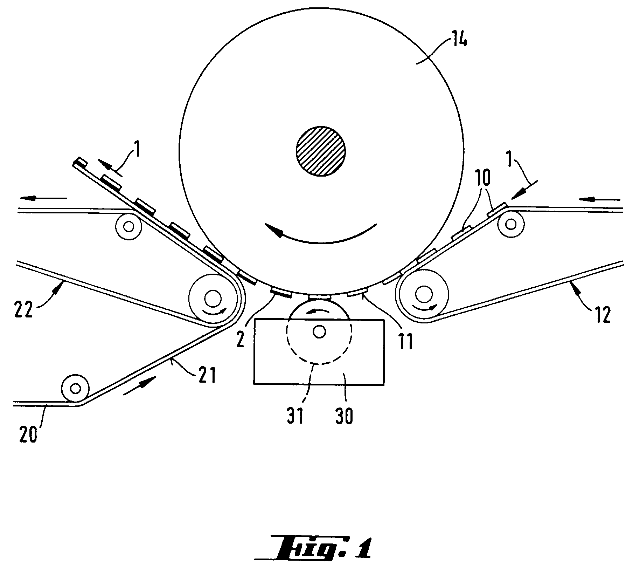

In figure 1, a process according to the present invention is shown wherein a series of separated pieces of a substrate, the separate being the whole sanitary napkins (10) but without panty fastening adhesive, is provided and conveyed in the machine direction (1) following the transport path of the substrate. A second, continuous material (20), the release paper, is also provided and conveyed. Figure 1 further shows a schematic roll printing unit (30) where adhesive (2) is provided to the first surface (11) of the sanitary napkins (10).

In the detailed cross-sectional figure 2 a continuous delivery of adhesive (2) to the first surface (11) is shown. The adhesive (2) is only transferred from roll printing unit (30) where the roll actually touches the sanitary napkin (10), i.e. in a discontinuous fashion.

In figure 2 it can be seen that the sanitary napkins (10) are conveyed in a machine direction (1) generally from the right hand side of the drawing guided by a material guiding system (12). From this guiding system the sanitary napkins (10) are transferred to another material guiding system (14) conveying them towards the roll printing unit (30).

The roll printing unit (30) comprises a rotating transport roll (31). The circular cross-section of the surface of rotating transport roll (31) describes the rotating path of rotating transport roll (31). Roll (31) in the receiving portion of the rotating path (34) dives into an adhesive bath (36). This is typically a bath of molten adhesive maintained inside the roll printing unit (30) at constant temperature by heating elements (not shown). Also roll (31) can and preferably does have internal heating elements such as hot oil or water channels to maintain the surface of transport roll (31) at a constant temperature.

The transport roll (31) is wetted by the molten adhesive in the receiving portion of rotating path (34). The roll surface continuously moves along the rotating path out of the bath of molten adhesive (36) and all excess adhesive is removed from the rotating transport roll (31) by scraper blade (35). Scraper blade (35) essentially rides on the surface of rotating transport roll (31). It can be kept at a constant nip by distance rings or other spacing mechanisms. It also can be provided with constant pressure onto the roll by spring loading scraper plate (35) or other means well known in the art of printing. Alternatively, the amount of adhesive taken up by rotating transport roll (31) can be controlled through the temperature and viscosity of the adhesive in the bath of molten adhesive (36) and the length of the receiving portion of the rotating path (34). Then a scraper plate (35) may not be necessary at all.

Following the path of the rotating transport roll (31) the delivery portion of the rotating path (33) is reached where the adhesive (2) is transferred from the transport roll to the first surface (11). In order for the adhesive (2) to transfer it is necessary that the adhesive strength between the adhesive (2) and the first surface (11) exceeds either the cohesive strength of the adhesive or the adhesive strength between the adhesive (2) and the surface of the transport roll (31) or both. Once the adhesive (2) is transferred to the first surface (11) it is further conveyed together with the sanitary napkin (10) to be joined with the release paper (20).

Material guiding system (14) provides the support for the separated sanitary napkin (10) during printing. Material guiding system (14) receives the individualized pieces, in the example sanitary napkins, from material guiding system (12). As can be seen in figure 2, material guiding system (14) holds the individualized pieces of the substrate (10) against gravitational forces. This can be achieved by any of the ways well-known in the art, particularly by vacuum suction in a direction opposite to the gravitational direction. A vacuum chamber (15) over which a screen transport surface of the guiding system (14) moves is indicated in figure 2.

In particular, material guiding system (14) has to hold the individualized pieces of substrate firmly attached at the end of the delivery portion of the rotating path since that is the place where the adhesive separates from the printing roll surface and is maintained on the first surface (11) of the substrate (10). This can for example be achieved by a increased vacuum suction at this point or other means well-known in the art. In respect to the required strength of holding the individualized pieces by guiding system (14) it is apparent that this must exceed the strength with which the adhesive holds the individualized pieces to the printing roll (31) or else the pieces would not continue the transport path as shown in figure 2 but stick to the adhesive transport roll (31) and cause a production interruption.

Release paper (20) is provided and introduced into the process according to the present invention by guidance system (22) as shown in figure 2. The distance between the point of joining it to the sanitary napkin by interfacing the adhesive (2) between first surface (11) and second surface (21) needs to be carefully set in order to ensure that the adhesive is still in a state to provide the desired adhesive attachment.

The pattern of the adhesive provided is continuous in machine direction (1) but can be defined as continuous or discontinuous by the printing surface of the rotating transport roll (31) in a direction (3) parallel to the rotation axis of the transport roll (31). The pattern preferably can be made of linear lines or strips of engravings or gravures (32). Most preferably, however, a full surface of engravings or gravures (32) is utilized.

The whole transport roll surface, including the engravings (32), will be wetted by the adhesive (2) in the receiving part of the rotating path of the transport roll (31). At the scraper plate (35) adhesive clinging to the roll surface outside the engravings (32) is removed and guided back into the bath of molten adhesive (36). Only the engravings remain filled with the adhesive which is transported on to the delivering portion of the rotation path of the rotating transport roll.

The "pattern of engravings" as used herein refers to the macroscopic area which is to be provided as adhesive area onto the first surface (11). This pattern is formed of a multitude of engravings (32) which are typically small enough to provide the whole area of adhesive with a layer of adhesive of about constant quantity per surface area. For example, patterns of engravings such as lines or strips are useful. Other adhesive applications may require other and additional patterns such a peripheral edge pattern to join the backsheet to the topsheet around the periphery of an absorbent core. The number of alternatives is unlimited but suitable patterns for the application will typically be easily identified by those skilled in the art.

As will be obvious for those skilled in the art, the shape, the depth, and density of individual engravings on the rotating transport roll surface in addition to the overall pattern of the engravings will be a critical parameter for the total amount of adhesive and basis weight supplied to first surface (11). Examples of the particular shape of engravings which have been found useful in the context of adhesive application for disposable absorbent articles such as sanitary napkins and pantiliners are shown in figures 3 through 14. The engravings are shown as a top plan view onto the rotating transport roll. For orientation a line is shown designated by reference numeral (3) which is parallel to the rotation axis of the rotating transport roll (31).

As can be seen in figures 1 or 2 this direction is typically perpendicular to the machine direction defined by the production path of the first material.

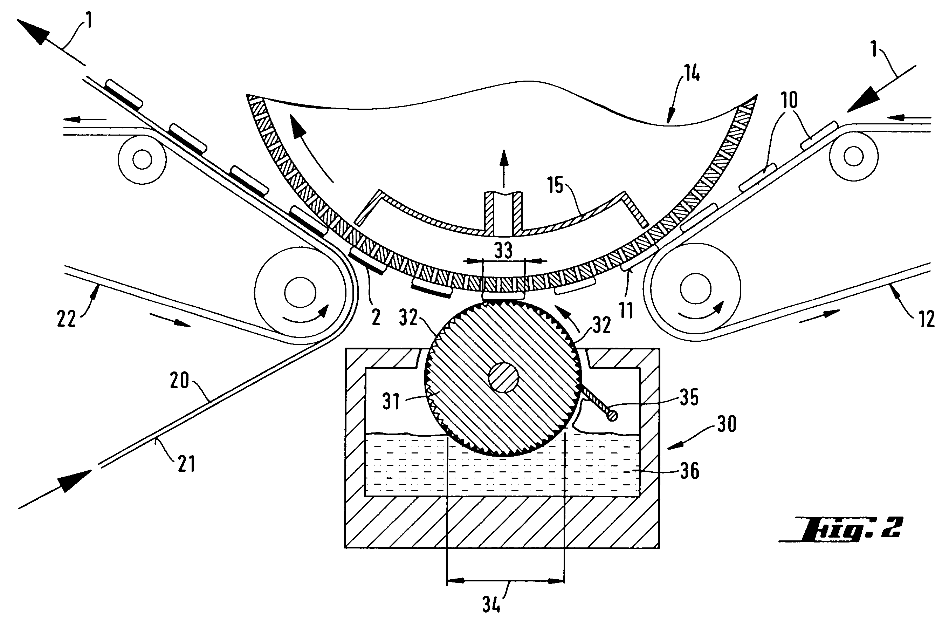

Figure 3 shows a square pyramidal shape with a depression angle of about 45° which can be seen in the cross-section shown in figure 4. Other angles such as 30°, 15° or steeper engravings at 60° or 75° can also be used. Figure 5 is similar to the square pyramidal engraving of figure 3, however, with a truncated central part of the pyramids which is shown in figure 6 in a cross-sectional view.

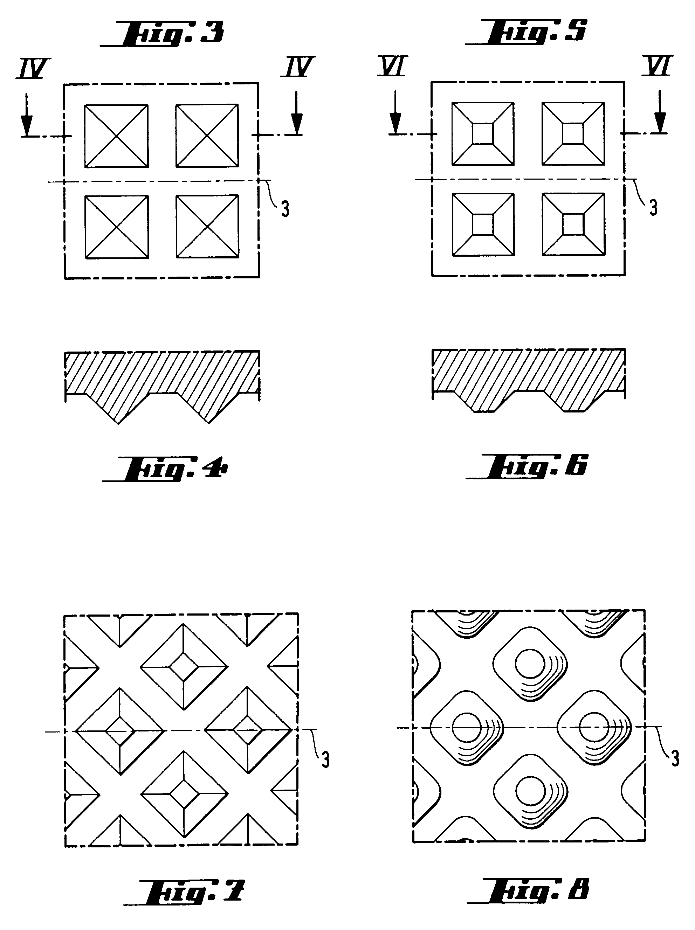

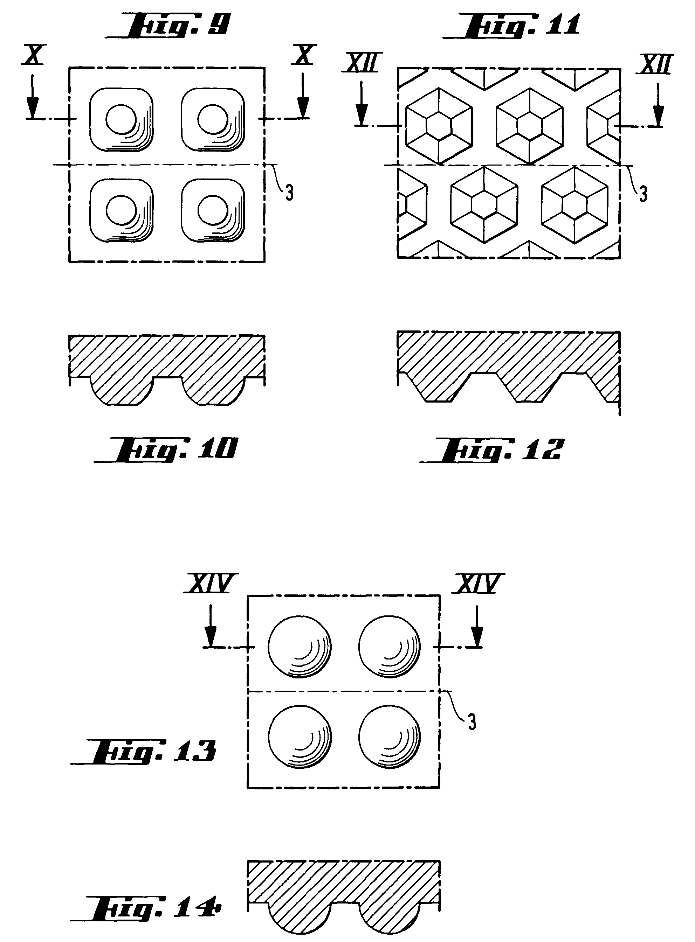

The direction of the individual engravings can of course alter relative to the rotation axes direction (3). This is seen when comparing figure 5 and figure 7 which shows the same truncated pyramid square engravings, however, with a change of their position relative to direction (3). Figure 8 shows a similar truncated engraving as figure 7, however, with the corners of the squares rounded. Figure 9 shows the same engraving as figure 8, however, with a different direction of the individual engravings. A cross-sectional representation of the engraving of figure 9 is shown in figure 10.

Figure 11 shows another alternative embodiment of the engraving in a truncated hexagonal pyramid. A cross-sectional representation of this engraving is shown in figure 12. Figure 13 and 14 show a round spherical engraving which is also an alternative.

The dimensions and density of these engravings can vary depending on the desired result of the adhesive printing step of the present invention. In the following preferable ranges for the engravings are given.

Each individual engraving can have a depth of from 0.01 mm for very small amounts of adhesive to be provided to 0.8 mm for the transport of large quantities of adhesive. An engraving size of 0.01 mm up to 2.5 mm as the side length for rectangular engravings or as the diameter for circular engraving has been found useful. For engravings which are neither square nor circular similar dimensions as those for square or circular engravings will be easily definable for those skilled in the art. The dimensions are of course taken on the surface of the rotating transport roll. A distance between engravings of 0.5 times to 50 times their depth has been found useful to provide an approximately even adhesive quantity distribution across the total engraved pattern.

The number of engravings per area depends on the individual engraving size. With increasing number of engravings and reducing size of individual engraving a more even adhesive delivery across the whole adhesive area will be achieved. Ultimately, a "full surface engraving" which parallels the total adhesive area can be utilised. This, however, would require distance rings or similar means to provide the scraper blade spacing to the roll.

An example of an engraving which has been successfully used in the context of the present invention is the engraving as shown in figure 6 with a depth of 0.067 mm, a size of 0.2 mm times 0.2 mm and 567 engravings per cm². These engravings were used to provide a panty fastening adhesive onto a polyethylene backsheet of a sanitary napkin according to the general description above. The panty fastening adhesive area followed the peripheral outline of the sanitary napkin in a so-called dog-bone-shape at a constant distance of approximately 4 mm.

Four alternative panty fastening adhesives were successfully tested at different temperatures. They were Savare LA203 which was applied at 130°C, Savare LA303 which was applied at 110°C, Fuller D3964ZP which was applied at 140°C and Coramelt 867 which was applied at 140°C. All application tests were conducted at surface speeds of up to 100 m/min. The second material providing the second surface was an usual siliconized release paper. Savare adhesives are available from Savare I.C. in Milan in Italy, Fuller adhesives are available from H.B. Fuller of Lueneburg in Germany and Coramelt adhesive is available from Koemmerling in Pirmasens in Germany.

## Claims

1. A process for providing an adhesive area to a first surface (11) of a substrate (10) for use in a disposable absorbent article, said adhesive area having a peripheral edge, said process comprising
a) providing a series of separated pieces of said substrate (10), each of said pieces of said substrate (10) comprising said first surface (11) said first surface (11) having a peripheral edge,
b) conveying said series of separated pieces of said substrate (10) in a machine direction (1),
c) providing an adhesive area onto said first surface on said pieces of said substrate (10), wherein said adhesive area is provided by continuous roll printing, thereby creating an adhesive area which has at least portions of its said peripheral edge coincide with said peripheral edge of said first surface (11).

2. A process according to claim 1 wherein all of said peripheral edge of said adhesive area coincides with said peripheral edge of said first surface (11).

3. A process according to claims 1 or 2 wherein said continuous roll printing comprising a rotating transport roll (31) for continuously transporting an adhesive (2), said roll (31) continuously receiving said adhesive (2) in a receiving portion of its rotation path (34) said receiving portion (34) being within a bath (36) of said adhesive (2), said roll (31) continuously transporting said adhesive (2) from said receiving portion of its rotation path (34) by rotating to a delivering portion of its rotation path (33) and said roll delivering said adhesive (2) to said first surface (11) in said delivering portion of its rotation path (33) discontinuously due to the discontinuous presence of said separated pieces.

4. A process according to any of the preceding claims wherein said adhesive area permanently joins said substrate (10) to said disposable absorbent article.

5. A process according to claims 1,2 or 3 wherein said disposable absorbent article is used in the undergarment of a user and comprises a panty fastening adhesive for realisably joining said article to the undergarment of a user of said article, said adhesive area providing said panty fastening adhesive.

6. A process according to claim 5 wherein said disposable absorbent article is prepared from a continuous substrate, said substrate being severed to provide separate absorbent articles and then said separated absorbent articles are provided with said panty fastening adhesive.

7. A process according to claim 6 wherein said disposable absorbent articles are sanitary napkins, catamenials or panty liners and comprise a topsheet, a backsheet and an absorbent core placed between said topsheet and said backsheet, said backsheet providing in use a garment facing surface of said articles, said first surface being said garment facing surface of said backsheet.

8. A process according to claim 6 or 7 comprising the additional steps of
d) providing a continuous strip of a releasable cover means;
e) placing said cover means onto said adhesive area on said separated absorbent articles, and
f) severing said cover means in order to provide individualised disposable articles.

9. A process according to claim 6 or 7 comprising the additional steps of
c) providing a continuous strip of a releasable cover means;
d) placing said cover means onto said adhesive area on said separated absorbent articles, and
e) storing said continuous strip of said cover means in order to provide a series of disposable articles which can be delaminated when needed.

## Revendications

1. Procédé pour fournir une zone adhésive à une première surface (11) d'un substrat (10) à utiliser dans un article absorbant jetable, ladite zone adhésive ayant un bord périphérique, ledit procédé comprenant les étapes consistant à :
a) fournir une série de pièces séparées dudit substrat (10), chacune desdites pièces dudit substrat (10) comprenant ladite première surface (11), ladite première surface (11) ayant un bord périphérique,
b) transporter lesdites séries de pièces séparées dudit substrat (10) dans le sens machine (1),
c) fournir une zone adhésive sur ladite première surface desdites pièces dudit substrat (10); dans lequel ladite zone adhésive est réalisée par impression continue au rouleau, ce qui engendre, ainsi, une zone adhésive qui a au moins des parties de son dit bord périphérique qui coïncident avec ledit bord périphérique de ladite première surface (11).

2. Procédé selon la revendication 1, dans lequel tout ledit bord périphérique de ladite zone adhésive coïncide avec ledit bord périphérique de ladite première surface (11).

3. Procédé selon l'une des revendications 1 ou 2, dans lequel ladite impression continue au rouleau comprend un rouleau rotatif de transport (31) pour transporter en continu un adhésif (2), ledit rouleau (31) recevant en continu ledit adhésif (2) dans une partie de réception de son trajet de rotation (34), ladite partie de réception (34) étant à l'intérieur d'un bain (36) dudit adhésif (2), ledit rouleau (31) transportant en continu ledit adhésif (2) depuis ladite partie de réception de son trajet de rotation (34) en tournant vers une partie de fourniture de son trajet de rotation (33), et ledit rouleau délivrant ledit adhésif (2) à ladite première surface (11) dans ladite partie de fourniture de son trajet de rotation (33), de manière discontinue en raison de la présence discontinue desdites pièces séparées.

4. Procédé selon l'une quelconque des revendications précédentes, dans lequel ladite zone adhésive réunit de manière permanente ledit substrat (10) audit article absorbant jetable.

5. Procédé selon l'une des revendications 1, 2 ou 3, dans lequel ledit article absorbant jetable est utilisé dans le sous-vêtement d'un utilisateur et comprend un adhésif d'attache de culotte pour réunir de manière précise ledit article au sous-vêtement d'un utilisateur dudit article. ladite zone adhésive fournissant ledit adhésif d'attache de culotte.

6. Procédé selon la revendication 5, dans lequel ledit article absorbant jetable est préparé à partir d'un substrat continu, ledit substrat étant coupé afin de fournir des articles absorbants séparés et, ensuite, lesdits articles absorbants séparés sont dotés dudit adhésif d'attache de culotte.

7. Procédé selon la revendication 6, dans lequel lesdits articles absorbants jetables sont des serviettes hygiéniques, des articles cataméniaux ou des garnitures de culotte, et comprennent une feuille de dessus, une feuille de fond et une âme absorbante placée entre ladite feuille de dessus et ladite feuille de fond, ladite feuille de fond fournissant, en utilisation, une surface faisant face au vêtement desdits articles, ladite première surface étant ladite surface faisant face au vêtement de ladite feuille de fond.

8. Procédé selon l'une des revendications 6 ou 7, comprenant les étapes supplémentaires consistant à :
d) fournir une bande continue d'un moyen de recouvrement libérable ;
e) placer ledit moyen de recouvrement sur ladite zone adhésive desdits articles absorbants séparés, et
f) couper ledit moyen de recouvrement afin de fournir des articles jetables distincts.

9. Procédé selon l'une des revendications 6 ou 7 comprenant les étapes supplémentaires consistant à :
c) fournir une bande continue d'un moyen de recouvrement libérable ;
d) placer ledit moyen de recouvrement sur ladite zone adhésive desdits articles absorbants séparés, et
e) stocker ladite bande continue dudit moyen de recouvrement afin de fournir une série d'articles jetables qui peuvent être déstratifiés si nécessaire.

## Patentansprüche

1. Ein Verfahren,um eine Klebezone auf eine erste Oberfläche (11) eines Substrates (10) zur Verwendung in einem wegwerfbaren absorbierenden Artikel anzubringen, wobei die genannte Klebezone eine peripheren Rand aufweist, wobei das genannte Verfahren umfaßt:
a) Beistellen einer Reihe von abgetrennten Stücken des genannten Substrates (10), wobei jedes der genannten Stücke dieses genannten Substrates (10) die genannte erste Oberfläche (11) aufweist, wobei die genannte erste Oberfläche (11) einen peripheren Rand umfaßt,
b) Befördern der genannten Reihe abgetrennter Stücke des genannten Substrates (10) in einer Maschinenrichtung (1),
c) Beistellen einer Klebezone auf die genannte erste Oberfläche an den genannten Stücken des genannten Substrates (10), bei welchem die genannte Klebezone durch kontinuierliches Walzendrucken beigestellt wird, wodurch eine Klebezone geschaffen wird, welche mindestens Abschnitte ihres genannten peripheren Randes koinzidierend mit dem genannten peripheren Rand der genannten ersten Oberfläche (11) aufweist,

2. Ein Verfahren nach Anspruch 1, bei welchem die Gesamtheit des genannten peripheren Randes der genannten Klebezone mit dem genannten peripheren Rand der genannten ersten Oberfläche (11) koinzidiert.

3. Ein Verfahren nach den Ansprüchen 1 oder 2, bei welchem das genannte kontinuierliche Walzendrucken eine rotierende Transportwalze (31) zum kontinuierlichen Transportieren eines Klebstoffs (2) umfaßt, wobei die genannte Walze (31) den genannten Klebstoff (2) in einem aufnehmenden Abschnitt ihres Rotationsweges (34) kontinuierlich aufnimmt, wobei der genannte aufnehmende Abschnitt (34) sich in einem Bad (36) des genannten Klebstoffs (2) befindet, wobei die genannte Walze (31) den genannten Klebstoff (2) vom genannten aufnehmenden Abschnitt ihres Rotationsweges (34) durch Rotieren zu einem abgebenden Abschnitt ihres Rotationsweges (33) kontinuierlich transportiert und die genannte Walze im genannten abgebenden Abschnitt ihres Rotationsweges (33) zufolge des diskontinuierlichen Vorhandenseins der genannten abgetrennten Stücke den genannten Klebstoff (2) auf die genannte erste Oberfläche (11) diskontinuierlich abgibt.

4. Ein Verfahren nach einem der vorhergehenden Ansprüche, bei welchem die genannte Klebezone das genannte Substrat (10) mit dem genannten wegwerfbaren absorbierenden Artikel permanent verbindet.

5. Ein Verfahren nach den Ansprüchen 1, 2 oder 3, bei welchen der genannte wegwerfbare absorbierende Artikel in der Unterwäsche eines Verwenders verwendet wird und einen Höschenbefestigungsklebstoff zum lösbaren Verbinden des genannten Artikels mit der Unterwäsche eines Verwenders des genannten Artikels umfaßt, wobei die genannte Klebezone den genannten Höschenbefestigungsklebstoff beistellt.

6. Ein Verfahren nach Anspruch 5, bei welchem der genannte wegwerfbare absorbierende Artikel aus einem kontinuierlichen Substrat hergestellt wird, wobei das genannte Substrat abgetrennt wird, um gesonderte absorbierende Artikel beizustellen, und dann die genannten gesonderten absorbierenden Artikel mit dem genannten Höschenbefestigungsklebstoff versehen werden.

7. Ein Verfahren nach Anspruch 6, bei welchem die genannten wegwerfbaren absorbierenden Artikel Hygienevorlagen, Monatshygiene-Einrichtungen oder Slipeinlagen sind und ein Deckblatt, ein Rückenblatt und einen absorbierenden Kern, welcher zwischen dem genannten Deckblatt und dem genannten Rückenblatt angeordnet ist, umfassen, wobei das genannte Rückenblatt bei Verwendung eine der Kleidung zugewandte Oberfläche der genannten Artikel beistellt, die genannte erste Oberfläche die genannte der Kleidung zugewandte Oberfläche des genannten Rückenblattes ist.

8. Ein Verfahren nach Anspruch 6 oder 7, welches die folgenden zusätzlichen Schritte aufweist:
d) Beistellen eines kontinuierlichen Streifens eines abnehmbaren Abdeckmittels;
e) Anordnen des genannten Abdeckmittels auf die genannte Klebezone an den genannten abgetrennten absorbierenden Artikeln, und
f) Abtrennen des genannten Abdeckmittels, um individualisierte wegwerfbare absorbierende Artikel beizustellen.

9. Ein Verfahren nach Anspruch 6 oder 7, welches folgende zusätzliche Schritte aufweist:
c) Beistellen eines kontinuierlichen Streifens eines abnehmbaren Abdeckmittels;
d) Anordnen des genannten Abdeckmittels auf die genannte Klebezone an den genannten abgetrennten absorbierenden Artikeln, und
e) Lagern des genannten kontinuierlichen Streifens des genannten Abdeckmittels, um eine Reihe von wegwerfbaren absorbierenden Artikeln beizustellen, welche bei Bedarf delaminiert werden können.
